# EUROPÄISCHE PATENTANMELDUNG

(11) **EP 4 205 722 A1**
(43) Veröffentlichungstag der Anmeldung: **05.07.2023**
(21) Anmeldenummer: 21218203.4
(22) Anmeldetag: 29.12.2021
(51) Int. Cl.: A61K 6/17, A61K 6/30, A61K 6/60, A61K 6/77, A61K 6/887, A61K 6/889, A61K 6/61, A61K 6/62

(54) **SELBSTHAFTENDER DENTALER KOMPOSITZEMENT MIT GUTER TRANSPARENZ**

(71) Anmelder: Ivoclar Vivadent AG, 9494 Schaan (LI)
(72) Erfinder: MOSZNER, Norbert, 9436 Triesen (LI); CATEL, Yohann, 9475 Rans (Sevelen) (CH); GIANASMIDIS, Alexandros, 9436 Balgach (CH); CATEL, Delphine, 9475 Rans (Sevelen) (CH); BOCK, Thorsten, 6800 Feldkirch (AT); GRABENBAUER, Barbara, 9445 Rebstein (CH); BROT, Andy, 9496 Balzers (LI); HALDNER, Tim, 9493 Mauren (LI)
(74) Vertreter: Uexküll & Stolberg

(57) **Zusammenfassung**

Radikalisch polymerisierbare Zusammensetzung, die mindestens ein saures, radikalisch polymerisierbares Monomer, mindestens einen Fluoroaluminiumsilikatglasfüllstoff und/oder röntgenopaken Glasfüllstoff und mindestens ein Maskierungsmittel enthält, das in fester Form vorliegt.

## Beschreibung

Die vorliegende Erfindung betrifft radikalisch polymerisierbare, selbsthaftende Komposite mit verbesserter Transparenz, die sich insbesondere als Dentalwerkstoffe eignen, z.B. als dentale Zemente, Füllungskomposite oder Verblendmaterialen sowie zur Herstellung von Inlays, Onlays oder Kronen.

Komposite werden im Dentalbereich hauptsächlich zur Herstellung von direkten und indirekten Füllungen, d.h. als direkte und indirekte Füllungskomposite, sowie als Zemente eingesetzt. Die polymerisierbare organische Matrix der Komposite besteht meistens aus einer Mischung von Monomeren, Initiator-Komponenten und Stabilisatoren. Als Monomere werden gewöhnlich Mischungen von Dimethacrylaten eingesetzt, die auch monofunktionelle und funktionalisierte Monomere enthalten können. Häufig eingesetzte Dimethacrylate sind 2,2-Bis[4-(2-hydroxy-3-methacryloyloxy-propyl)phenyl]propan (Bis-GMA), 1,6-Bis-[2-methacryloyloxyethoxycarbonylamino]-2,2,4-trimethylhexan (UDMA), die eine hohe Viskosität haben und Polymerisate mit sehr guten mechanischen Eigenschaften und geringem Polymerisationsschrumpf ergeben. Als reaktive Verdünner finden vor allem Triethylenglycoldimethacrylat (TEGDMA), 1,10-Decandioldimethacrylat (D₃MA) oder Bis(3-methacryloyloxy-methyl)tricyclo-[5.2.1.0^{2,6}]decan (DCP) Anwendung. Monofunktionelle Methacrylate, wie z.B. p-Cumylphenoxyethylenglycolmethacrylat (CMP-1E), eignen sich ebenfalls zur Reduzierung der Viskosität und bewirken darüber hinaus eine Verringerung der Netzwerkdichte und einen erhöhten Doppelbindungsumsatz.

Zur Herstellung selbsthaftender Komposite werden stark saure Haftmonomere eingesetzt, wie z.B. 10-Methacryloyloxydecyldihydrogenphosphat (MDP), das die Zahnhartsubstanz ätzt und durch Ionenbeziehung zu einer Haftung auf Schmelz/Dentin führt. Haftmonomere verleihen Kompositen selbsthaftende Eigenschaften und ermöglichen damit den Einsatz der Komposite ohne eine Vorbehandlung der Zahnhartsubstanz mit einem Schmelz-Dentin-Adhäsiv, was deren Verwendung besonders attraktiv macht.

Neben der organischen Matrix enthalten Komposite einen oder mehrere Füllstoffe, die meist mit einem polymerisationsfähigen Haftvermittler, wie z.B. 3-Methacryloyloxypropyltrimethoxysilan, oberflächenmodifiziert sind. Füllstoffe verbessern die mechanischen Eigenschaften (Festigkeit, Elastizitätsmodul, Abrasionsfestigkeit) und die Verarbeitungseigenschaften (Pastenkonsistenz, Stopfbarkeit) der Werkstoffe und verleihen ihnen Röntgenopazität.

Problematisch ist, dass saure Haftmonomere mit Füllstoffen häufig nachteilige Wechselwirkungen eingehen. Beispielsweise werden die sauren Haftmonomere durch die Bildung unlöslicher Salze an die Oberfläche der Füllstoffe gebunden, oder sie bilden bei der Lagerung mit aus den Füllstoffen herausgelösten Ionen schwerlösliche Salze. Dies führt zu einer deutlichen Verringerung der Haftmonomerkonzentration in der Harzmatrix, was mit einer Verminderung oder sogar einem Verlust der Hafteigenschaften des Zements verbunden ist. Komposite mit sauren Haftmonomeren haben daher nur eine begrenzte Lagerstabilität.

Die Aushärtung methacrylatbasierender Dentalmaterialien erfolgt durch radikalische Polymerisation, wobei je nach Anwendungsgebiet radikalische Photoinitiatoren, thermische Initiatoren oder Redox-Initiatorsysteme eingesetzt werden. Dualhärtende Systeme enthalten eine Kombination von Photoinitiatoren und Redoxinitiatoren.

Kompositzemente enthalten in der Regel Redoxsysteme, weil diese auch dann eine ausreichende Härtung gewährleisten, wenn eine Lichthärtung wegen einer unzureichenden Durchstrahlung nicht möglich ist. Meist werden Redoxinitiatorsysteme eingesetzt, die auf einer Mischung von Dibenzoylperoxid (DBPO) mit tertiären aromatischen Aminen, wie z.B. N,N-Diethanol-p-toluidin (DEPT), N,N-Dimethyl-sym.-xylidin (DMSX) oder N,N-Diethyl-3,5-di-tert.-butylanilin (DABA) basieren. Da die Radikalbildung bei DBPO/Amin-basierenden Redoxinitiatorsystemen durch starke Säuren und damit auch durch stark saure Haftmonomere sehr beeinträchtigt wird, werden bevorzugt Cumolhydroperoxid-haltige Redoxinitiatorsysteme in Kombination mit Thioharnstoffen, wie z.B. Acetylthioharnstoff, verwendet.

Um eine ausreichende Lagerstabilität der Redoxinitiatoren zu gewährleisten, werden Redox-Initiatorsystem-basierende Materialien meist als sog. 2-Komponenten-Systeme (2K) eingesetzt, wobei das Oxydationsmittel (Peroxid- oder Hydroperoxid) und das Reduktionsmittel (Amine, Sulfinsäuren, Barbiturate, Thioharnstoffe etc.) in voneinander getrennte Komponenten eingearbeitet werden. Diese werden erst kurz vor der Anwendung miteinander gemischt. Zum Mischen werden zunehmend Doppelschubspritzen verwendet, die getrennte zylindrische Kammern zur Aufnahme der Komponenten aufweisen. Die Komponenten werden mit zwei miteinander verbundenen Kolben gleichzeitig aus den Kammern herausgedrückt und in einer Düse miteinander gemischt. Um möglichst homogene Mischungen zu erhalten, ist es vorteilhaft, die Komponenten in etwa gleich großen Volumenanteilen miteinander zu mischen.

Herkömmliche Befestigungszemente, wie z.B. ZnO-Eugenol-Zemente, Zinkphosphat-Zemente, Glasionomer-Zemente (GIZ) und harzmodifizierte Glasionomer-Zemente (Resin Modified Glass Ionomer Cements: RMGI) eigenen sich nicht zur Anwendung mit Doppelschubspritzen, weil sie eine Pulverkomponente enthalten, was ein Mischen der Komponenten erheblich erschwert. Außerdem haben Glasionomerzemente nur eine geringe Transparenz und relativ schlechte mechanische Eigenschaften.

Konventionelle Glasionomer-Zemente (GIZ) enthalten als flüssige Komponente eine wässrige Lösung einer hochmolekularen Polyacrylsäure (PAA, zahlenmittlere Molmasse größer 30 000 g/mol) oder eines Copolymers vergleichbarer Molmasse aus Acrylsäure und Itaconsäure und als Pulverkomponente ein Calcium-Fluor-Aluminium-Glas. Sie härten nach dem Mischen der Komponenten durch eine rein ionische lonomerbildung aus. Nachteilig an Glasionomerzementen sind ihre geringe Transparenz und ihre schlechten mechanischen Eigenschaften.

Harzmodifizierte Glasionomer-Zemente (Resin Modified Glass Ionomer Cements: RMGI) enthalten zusätzlich hydrophile Monomere, wie z.B. 2-Hydroxyethylmethacrylat (HEMA). Ihre Aushärtung erfolgt sowohl durch eine Säure-Base-Reaktion als auch durch radikalische Polymerisation. Sie zeichnen sich gegenüber herkömmlichen GIZ durch eine verbesserte Biegefestigkeit aus.

Die US 2004/0048226A1 offenbart eine Methode zur Zahnwurzelbehandlung, bei der eine EDTA-haltige Flüssigkeit als Reinigungs- und Sterilisationslösung verwendet wird.

Die US 8,648,062 B2 offenbart eine EDTA-haltige Zusammensetzung zur Spülung von präparierten Zahnwurzelkanälen. Die Zusammensetzung soll das gleichzeitige Entfernen der Schmierschicht und Desinfizieren ermöglichen.

In der US 2014/0294742 A1 werden peroxidhaltige Lösungen zur Behandlung von dentalen Belägen beschrieben, die als Stabilisator EDTA oder ein EDTA-Salz enthalten können.

Die JP 11060428A offenbart ein dentales Adhäsiv, das eine wässrige Säurelösung zur Vorbehandlung der Zahnoberfläche enthält. Geeignete Säuren sind EDTA, Phosphorsäure und Zitronensäure.

Die EP 3 045 160 A1 betrifft dentale Komposite auf der Basis von (Meth)acrylatmonomeren und Füllstoff, die als Initiator für die radikalische Polymerisation ein Barbiturat in Kombination mit einer Peroxidverbindung enthalten. Die Komposite enthalten zusätzlich Aminocarbonsäure-Chelatbildner, wie z.B. EDTA oder Salze davon, die eine Reaktion der Barbitursäurederivate mit Metallionen verhindern und so die Lagerstabilität der Werkstoffe verbessern sollen.

Die DE 10 2005 022 172 A1 offenbart polymerisierbare EDTA-Derivate, in denen EDTA kovalent mit ethylenisch ungesättigten Monomeren verknüpft ist. Die EDTA-Derivate werden bei der Polymerisation durch Integration in die organische Matrix von Dentalmaterialien immobilisiert und sollen die die Haftung von dentalen Adhäsiven an Zähnen verbessern.

Die EP 2 065 363 A1 offenbart Dentalwerkstoffe auf der Basis hydrolysestabiler Alky-lendiamin-N,N,N',N'-tetraessigsäure-(meth)acrylamide, die eine gute Wasserlöslichkeit aufweisen und die Haftung der Werkstoffe auf Zahnschmelz und Dentin verbessern.

Der Erfindung liegt die Aufgabe zugrunde, lagerstabile, selbsthaftende dentale Komposite mit guter Transparenz und guten mechanischen Eigenschaften bereitzustellen, die sich als 2-Komponentensysteme gut mit Doppelschubspritzen mischen und applizieren lassen. Die Komposite sollen sich insbesondere als dentale Befestigungszemente eignen.

Diese Aufgabe wird durch füllstoffhaltige, radikalisch polymerisierbare Zusammensetzungen gelöst, die mindestens ein saures, radikalisch polymerisierbares Monomer, mindestens einen Fluoroaluminiumsilikatglasfüllstoff und/oder röntgenopaken Glasfüllstoff und mindestens ein festes Maskierungsmittel enthalten. Es wurde überraschend gefunden, dass Maskierungsmittel, die zumindest teilweise in ungelöster Form vorliegen, eine deutliche Erhöhung der Lagerstabilität von Zusammensetzungen bewirken, die saure Monomere und mindestens einen Fluoroaluminiumsilikatglasfüllstoff und/oder röntgenopaken Glasfüllstoff enthalten.

Erfindungsgemäß bevorzugte Maskierungsmittel sind Ethylendiamintetraessigsäure (EDTA) und deren Dinatrium-Salz (Dinatriumethylendiamintetraacetat), Nitrilotriessigsäure, Diethylentriaminpentaessigsäure, Tetranatriumiminodisuccinat und das Trinatriumsalz der Methylglycindiessigsäure. Besonders bevorzugt ist EDTA.

Das oder die Maskierungsmittel werden vorzugsweise in einer Gesamtmenge von 0,5 bis 6,0 Gew.-%, besonders bevorzugt 0,7 bis 5 Gew.-% und ganz besonders bevorzugt 1,0 bis 4,0 Gew.-% zugegeben. Alle Prozentangaben hierin beziehen sich, wenn nicht anders angegeben, auf die Gesamtmasse der Zusammensetzung.

Das oder die Maskierungsmittel werden vorzugsweise in partikulärer Form eingesetzt und verhalten sich dann praktisch wie ein Füllstoff, was im Hinblick auf die die mechanischen Eigenschaften vorteilhaft ist. Bevorzugt sind Partikel mit einer mittleren Größe (D50-Wert) von 300 bis 350 µm, besonders bevorzugt von 200 bis 250 µm und ganz besonders bevorzugt von 100 bis 170 µm. Gemäß einer besonders bevorzugten Ausführungsform der Erfindung liegt der D10-Wert der Partikel in einem Bereich von 130 bis 150 µm, besonders bevorzugt 80 bis 100 µm und ganz besonders bevorzugt 70 bis 55 µm. Der D50-Wert gibt die mittlere Partikelgröße an. D50 bedeutet, dass 50% der Partikel kleiner sind als der angegebene Wert. Ein weiterer wichtiger Parameter ist der D10-Wert als Maß für die kleinsten Partikel. D10 bedeutet, dass 10% der Partikel kleiner als der angegebene Wert sind.

Wenn nicht anders angegeben, handelt es sich bei allen Partikelgrößen hierin um volumengemittelte Partikelgrößen (D50-Werte), d.h. 50 % des Gesamtvolumens, das alle Partikel gemeinsam besitzen, ist in Partikeln enthalten, die einen Durchmesser kleiner als den angegebene Wert aufweisen. Die D10-Werte beziehen sich ebenfalls auf den volumetrischen Durchmesser.

Die Partikelgrößenbestimmung im Bereich von 0,1 µm bis 1000 µm erfolgt vorzugsweise mittels statischer Lichtstreuung (SLS), beispielsweise mit einem statischen Laserstreuungs-Partikelgrößen-Analysator LA-960 (Horiba, Japan) oder mit einem Microtrac S100 Partikelgrößen-Analysator (Microtrac, USA). Hierbei werden als Lichtquellen eine Laser-Diode mit einer Wellenlänge von 655 nm und eine LED mit einer Wellenlänge von 405 nm verwendet. Der Einsatz von zwei Lichtquellen mit unterschiedlichen Wellenlängen ermöglicht die Vermessung der gesamten Partikelgrößenverteilung einer Probe in nur einem Messungsdurchgang, wobei die Messung als Nassmessung durchgeführt wird. Hierzu wird eine wässrige Dispersion des Füllstoffs hergestellt und deren Streulicht in einer Durchflusszelle gemessen. Die Streulichtanalyse zur Berechnung von Partikelgröße und Partikelgrößenverteilung erfolgt gemäß der Mie-Theorie nach DIN/ISO 13320. Die Messung der Partikelgröße in einem Bereich von 1 nm bis 0,1 µm erfolgt vorzugsweise durch dynamische Lichtstreuung (DLS) von wässrigen Partikeldispersionen, vorzugsweise mit einem He-Ne-Laser mit einer Wellenlänge von 633 nm, bei einem Streuwinkel von 90° und bei 25°C, z.B. mit einem Malvern Zetasizer Nano ZS (Malvern Instruments, Malvern UK).

Im Fall aggregierter und agglomerierter Partikel kann die Primärteilchengröße anhand von TEM-Aufnahmen bestimmt werden. Die Transmissionselektronenmikroskopie (TEM) wird vorzugsweise mit einem Philips CM30 TEM bei einer Beschleunigungsspannung von 300 kV durchgeführt. Für die Probenvorbereitung werden Tropfen der Partikeldispersion auf ein 50 Å dickes Kupfergitter (Maschenweite 300 Mesh) aufgebracht, das mit Kohlenstoff beschichtet ist, und im Anschluss das Lösungsmittel verdampft. Die Partikel werden ausgezählt und der arithmetische Mittelwert berechnet.

Die erfindungsgemäßen Zusammensetzungen enthalten mindestens ein radikalisch polymerisierbares Monomer, vorzugsweise ein oder mehrere mono- und/oder polyfunktionelle Monomere. Unter polyfunktionellen Monomeren werden Verbindungen mit zwei oder mehr, vorzugsweise 2 bis 4 und insbesondere 2 radikalisch polymerisierbaren Gruppen verstanden. Monofunktionelle Monomere weisen dementsprechend nur eine radikalisch polymerisierbare Gruppe auf. Polyfunktionelle Monomere haben vernetzende Eigenschaften und werden daher auch als Vernetzermonomere bezeichnet. Bevorzugte radikalisch polymerisierbare Gruppen sind (Meth)acrylat-, (Meth)acrylamid- und Vinylgruppen.

Erfindungsgemäß wird zwischen säuregruppenhaltigen Monomeren und Monomeren unterschieden, die keine Säuregruppen enthalten. Die erfindungsgemäßen Zusammensetzungen enthalten mindestens ein Monomer ohne Säuregruppen und mindestens ein Monomer und/oder Oligomer mit Säuregruppen. Erfindungsgemäß sind Zusammensetzungen bevorzugt, die Monomere mit und Monomere ohne Säuregruppe in einem Gewichtsverhältnis von 1:5 bis 1:36, besonders bevorzugt von 1:6 bis 1:25 und ganz besonders bevorzugt von 1:7 bis 1:20 enthalten.

### Monomere ohne Säuregruppe

Bevorzugt sind Zusammensetzungen, die mindestens ein (Meth)acrylat enthalten, besonders bevorzugt mindestens ein monofunktionelles oder polyfunktionelles Methacrylat und ganz besonders bevorzugt mindestens ein monofunktionelles oder difunktionelles Methacrylat oder eine Mischung davon.

Bevorzugte monofunktionelle (Meth)acrylate sind Benzyl-, Tetrahydrofurfuryl- oder Isobornyl(meth)acrylat, p-Cumyl-phenoxyethylenglycolmethacrylat (CMP-1E) und 2-([1,1'-Biphenyl]-2-oxy)ethylmethacrylat (MA-836), Tricyclodecanmethyl(meth)-acrylat, 2-(2-Biphenyloxy)ethyl(meth)acrylat. Besonders geeignet sind CMP-1E und MA-836.

Gemäß einer Ausführungsform enthalten die erfindungsgemäßen Zusammensetzungen vorzugsweise mindestens ein funktionalisiertes monofunktionelles (Meth)acrylat. Unter funktionalisierten Monomeren werden solche Monomere verstanden, die neben mindestens einer radikalisch polymerisierbaren Gruppe mindestens eine funktionelle Gruppe tragen, vorzugsweise eine Hydroxylgruppe. Bevorzugte funktionalisierte Mono(meth)acrylate sind 2-Hydroxyethyl- und Hydroxyethylpropyl(methacrylat) sowie 2-Acetoxyethylmethacrylat. Besonders bevorzugt ist Hydroxyethylmethacrylat. Die unten genannten säuregruppenhaltigen Monomere sind keine funktionalisierten Monomere im Sinne der Erfindung.

Bevorzugte di- und polyfunktionelle (Meth)acrylate sind Bisphenol-A-dimethacrylat, Bis-GMA (ein Additionsprodukt aus Methacrylsäure und Bisphenol-A-diglycidylether), ethoxy- oder propoxylierte Bisphenol-A-dimethacrylate, wie z.B. das Bisphenol-A-dimethacrylat SR-348c (Sartomer) mit 3 Ethoxygruppen oder 2,2-Bis[4-(2-methacryl-oyloxypropoxy)phenyl]propan, Urethane aus 2-(Hydroxymethyl)acrylsäuremethylester und Diisocyanten, wie 2,2,4-Trimethylhexamethylendiisocyanat oder Isophorondiisocyanat, UDMA (ein Additionsprodukt aus 2-Hydroxyethylmethacrylat und 2,2,4-Trimethylhexamethylen-1,6-diisocyanat), Tetramethylxylylendiurethanethylendi(meth)acrylat bzw. Tetramethylxylylendiurethan-2-methylethylenglykoldi(meth)-acrylat (V380), Di-, Tri- oder Tetraethylenglycoldimethacrylat, Trimethylolpropantrimethacrylat, Pentaerythrittetramethacrylat sowie Glycerindi- und -trimethacrylat, 1,4-Butandioldimethacrylat, 1,10-Decandioldimethacrylat (D₃MA), Bis(methacryl-oyloxymethyl)tricyclo-[5.2.1.0^{2,6}]decan (DCP), Polyethylenglycol- oder Polypropylenglycoldimethacrylate, wie z.B. Polyethylenglycol-200-dimethacrylat oder Polyethylenglycol-400-dimethacrylat (PEG-200- oder PEG-400-DMA) oder 1,12-Dodecandioldimethacrylat. Besonders bevorzugt sind Bis-GMA, UDMA, V-380, Triethylenglycoldimethacrylat (TEGDMA) und PEG-400-DMA (NK-Ester 9G).

Das Monomer Tetramethylxylylendiurethanethylenglykoldi(meth)acrylat bzw. Tetra-methylxylylendiurethan-2-methylethylenglykoldi(meth)acrylat (V380), weist die folgende Formel auf: 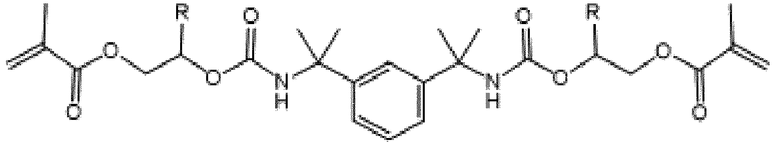 V380 (R = H, CH₃)

In der gezeigten Formel sind die Reste R unabhängig voneinander H oder CH₃, wobei die Reste die gleiche Bedeutung oder unterschiedliche Bedeutungen haben können. Vorzugsweise wird eine Mischung eingesetzt, die Moleküle, in denen beide Reste H sind, Moleküle, in denen beide Reste CH₃ sind, und Moleküle enthält, in denen ein Rest H und der andere Rest CH₃ ist, wobei das Verhältnis von H zu CH₃ vorzugsweise 7:3 ist. Eine solche Mischung ist beispielsweise durch Reaktion von 1,3-Bis(1-isocyanato-1-methylethyl)benzol mit 2-Hydroxypropylmethacrylat und 2-Hydroxyethylmethacrylat erhältlich.

Weitere bevorzugte difunktionelle Monomere sind radikalisch polymerisierbare Pyrrolidone, wie z.B. 1,6-Bis(3-vinyl-2-pyrrolidonyl)-hexan, oder kommerziell zugängliche Bisacrylamide wie Methylen- oder Ethylenbisacrylamid, sowie Bis(meth)acrylamide, wie z.B. N,N-Diethyl-1,3-bis(acrylamido)-propan, 1,3-Bis(methacrylamido)-propan, 1,4-Bis(acrylamido)-butan oder 1,4-Bis(acryloyl)-piperazin, die durch Umsetzung aus den entsprechenden Diaminen mit (Meth)acrylsäurechlorid synthetisiert werden können. Besonders bevorzugt ist N,N'-Diethyl-1,3-bis(acrylamido)propan (V-392). Diese Monomere zeichnen sich durch eine hohe Hydrolysestabilität aus.

### Säuregruppenhaltige Monomere und Oligomere

Die erfindungsgemäßen Zusammensetzungen enthalten mindestens ein saures, radikalisch polymerisierbares Monomer und/oder mindestens ein saures Oligomer. Unter sauren Monomeren und Oligomeren werden Monomere bzw. Oligomere verstanden, die mindestens eine Säuregruppe enthalten, vorzugsweise eine Phosphorsäureester-, Phosphonsäure- oder Carboxygruppe, besonders bevorzugt sind Phosphorsäureester. Saure Monomere und Oligomere werden hierin auch als Haftkomponente bzw. Haftmonomere oder Haftoligomere bezeichnet.

Bevorzugte säuregruppenhaltige Monomere sind Phosphorsäureester- und Phosphonsäuremonomere. Besonders bevorzugte sind 2-Methacryloyloxyethylphenyl-hydrogenphosphat, 10-Methacryloyloxydecyl-dihydrogenphosphat (MDP) Glycerindimethacrylatdihydrogenphosphat oder Dipentaerythritpentamethacryloyloxyphosphat. 4-Vinylbenzylphosphonsäure, 2-[4-(Dihydroxyphosphoryl)-2-oxa-butyl]-acrylsäure oder hydrolysestabile Ester, wie z.B. 2-[4-(Dihydroxyphosphoryl)-2-oxa-butyl]-acrylsäure-2,4,6-trimethylphenylester. Ganz besonders bevorzugt sind MDP, 2-Methacryloyloxyethylphenyl-hydrogenphosphat und Glycerindimethacrylatdihydrogenphosphat.

Weitere bevorzugte säuregruppenhaltige Monomere sind COOH-gruppenhaltige polymerisationsfähige Monomere. Besonders bevorzugt sind 4-(Meth)acryloyl-oxyethyltrimellitsäureanhydrid, 10-Methacryloyloxydecylmalonsäure, N-(2-Hydroxy-3-methacryloyloxypropyl)-N-phenylglycin und 4-Vinylbenzoesäure.

Unter Oligomeren werden Polymere mit einem Polymerisationsgrad Pₙ von 2 bis 100 verstanden (Pₙ = Mₙ / Mᵤ; Mₙ: zahlenmittlere Polymermolmasse, Mᵤ: Molmasse der Monomereinheit). Saure, radikalisch polymerisierbare Oligomere weisen mindestens eine Säuregruppe, vorzugsweise Carboxygruppe, und mindestens eine radikalisch polymerisierbare Gruppe, vorzugsweise mindestens eine (Meth)acrylat- und insbesondere mindestens eine Methacrylatgruppe, auf.

Erfindungsgemäß bevorzugte säuregruppenhaltige Oligomere sind oligomere Carbonsäuren, wie z.B. Polyacrylsäure, vorzugsweise mit einer zahlenmittleren Molmasse Mₙ kleiner 7200 g/mol, bevorzugt kleiner 7000 g/mol und besonders bevorzugt kleiner 6800 g/mol, wobei Mₙ bevorzugt in einem Bereich von 800 bis 7200 g/mol, besonders bevorzugt von 500 bis 7000 g/mol und ganz besonders bevorzugt von 500 bis 6800 g/mol liegt. Besonders bevorzugt sind oligomere Carbonsäuren mit (Meth)acrylatgruppen. Diese sind z.B. durch Umsetzung von oligomerer Polyacrylsäure mit Glycidylmethacrylat oder 2-Isocyanatoethylmethacrylat erhältlich.

Wenn nicht anders angegeben, handelt es sich hierin bei der Molmasse von Oligomeren und Polymeren um die zahlenmittlere Molmasse, deren Absolutwerte man mit den bekannten Methoden der Gefrierpunktserniedrigung (Kryoskopie), der Siedepunktserhöhung (Ebullioskopie) oder aus der Erniedrigung des Dampfdrucks (Dampfdruckosmometrie) bestimmen kann. Vorzugsweise wird die zahlenmittlere Molmasse von Oligomeren und Polymeren mittels Gel-Permeations-Chromatographie (GPC) bestimmt. Dabei handelt es sich um eine Relativmethode bei der die Moleküle aufgrund ihrer Größe, genauer gesagt aufgrund ihres hydrodynamischen Volumens, getrennt werden. Die Bestimmung der absoluten Molmasse erfolgt durch Kalibrierung mit bekannten Standards.

Die erfindungsgemäßen Zusammensetzungen enthalten darüber hinaus vorzugsweise auch Wasser. Es wurde gefunden, dass ein Wassergehalt von 1 bis 7 Gew.-%, besonders bevorzugt 1 bis 5 Gew.-%, jeweils bezogen auf die Gesamtmasse der Zusammensetzung, eine Verbesserung der Haftwirkung an Dentin und Zahnschmelz bewirkt, aber nicht zu einem vollständigen Auflösen des Maskierungsmittels führt.

Die erfindungsgemäßen Zusammensetzungen enthalten außerdem mindestens einen Initiator zur Initiierung der radikalischen Polymerisation, vorzugsweise einen Photoinitiator. Bevorzugte Photoinitiatoren sind Benzophenon, Benzoin sowie deren Derivate, α-Diketone oder deren Derivate, wie 9,10-Phenanthrenchinon, 1-Phenyl-propan-1,2-dion, Diacetyl und 4,4'-Dichlorbenzil. Besonders bevorzugt werden Campherchinon (CC) und 2,2-Dimethoxy-2-phenyl-acetophenon und ganz besonders bevorzugt α-Diketone in Kombination mit Aminen als Reduktionsmittel, wie z.B. 4-(Dimethylamino)-benzoesäureethylester (EDMAB), N,N-Dimethylaminoethylmethacrylat, N,N-Dimethyl-sym.-xylidin oder Triethanolamin verwendet. Weiter bevorzugt sind Norrish-Typ-I-Photoinitiatoren, vor allem Acyl- oder Bisacylphosphinoxide und ganz besonders Monoacyltrialkyl-, Diacyldialkylgermanium- und Tetraacylgermanium-Verbindungen, wie z.B. Benzoyltrimethylgerman, Dibenzoyldiethylgerman, Bis(4-methoxybenzoyl)diethylgerman (Ivocerin^{®}), Tetrabenzoylgerman oder Tetrakis(o-methylbenzoyl)german. Dabei lassen sich auch Mischungen der verschiedenen Photoinitiatoren einsetzen, wie z.B. Bis(4-methoxybenzoyl)diethylgerman oder Tetrakis(o-methylbenzoyl)german in Kombination mit Campherchinon und 4-Dimethylaminobenzoesäureethylester.

Weiter bevorzugt sind Zusammensetzungen, die einen Redoxinitiator zur Initiierung der radikalischen Polymerisation enthalten, vorzugsweise einen Redoxinitiator auf der Basis eines Oxidationsmittels und eines Reduktionsmittel. Bevorzugte Oxidationsmittel sind Peroxide und insbesondere Hydroperoxide. Ein besonders bevorzugtes Peroxid ist Benzoylperoxid. Bevorzugte Hydroperoxide sind die in EP 3 692 976 A1 offenbarten geruchsarmen Cumolhydroperoxid-Derivate, die in EP 21315089.9 offenbarten oligomeren CHP-Derivate und insbesondere 4-(2-Hydroperoxypropan-2-yl)phenylpropionat und Cumolhydroperoxid (CHP).

Bevorzugte Reduktionsmittel zur Kombination mit Peroxiden sind tertiäre Amine, wie N,N-Dimethyl-p-toluidin, N,N-Dihydroxyethyl-p-toluidin, p-Dimethylaminobenzoesäureethylester oder andere aromatische Dialkylamine, Ascorbinsäure, Sulfinsäuren, Thiole und/oder Hydrogensilane.

Bevorzugte Reduktionsmittel zur Kombination mit Hydroperoxiden sind Thioharnstoffderivate, insbesondere die in der EP 1 754 465 A1 in Absatz [0009] aufgeführten Verbindungen. Besonders bevorzugt sind Methyl-, Ethyl-, Allyl-, Butyl-, Hexyl-, Octyl-, Benzyl-, 1,1,3-Trimethyl-, 1,1-Diallyl, 1,3-Diallyl-, 1-(2-Pyridyl-2-thioharnstoff, Acetyl-, Propanoyl-, Butanoyl-, Pentanoyl-, Hexanoyl-, Heptanoyl-, Octanoyl, Nonanoyl, Decanoyl, Benzoylthioharnstoff und Mischungen davon. Ganz besonders bevorzugt sind Acetyl-, Allyl-, Pyridyl- und Phenylthioharnstoff sowie Hexanoylthioharnstoff und Mischungen davon sowie polymerisationsfähige ThioharnstoffDerivate, wie z.B. *N*-(2-Methacryloyloxyethoxysuccinoyl)-thioharnstoff und *N*-(4-Vinylbenzoyl)-thioharnstoff). Außerdem kann vorteilhaft eine Kombination von einem oder mehreren der genannten Thioharnstoffderivate mit einem oder mehreren Imidazolen eingesetzt werden. Bevorzugte Imidazole sind 2-Mercapto-1-methylimidazol oder 2-Mercaptobenzimidazol.

Die erfindungsgemäßen Zusammensetzungen können neben mindestens einem Hydroperoxid und mindestens einem Thioharnstoffderivat außerdem zusätzlich mindestens eine Übergangsmetallverbindung zur Beschleunigung der Härtung enthalten. Erfindungsgemäß geeignete Übergangsmetallverbindungen sind insbesondere Verbindungen, die sich von Übergangsmetallen ableiten, die mindestens zwei stabile Oxidationsstufen haben. Besonders bevorzugt sind Verbindungen der Elemente Kupfer, Eisen, Kobalt, Nickel und Mangan. Diese Metalle weisen die folgenden stabilen Oxydationsstufen auf: Cu(I)/Cu(II), Fe(II)/Fe(III), Co(II)/Co(III), Ni(II)/Ni(III), Mn(II)/Mn(III). Zusammensetzungen, die mindestens eine Kupferverbindung enthalten, sind besonders geeignet. Die Übergansmetallverbindungen werden vorzugsweise in katalytische Mengen eingesetzt, besonders bevorzugt in einer Menge von 10 bis 200 ppm. Diese führen zu keiner Verfärbungen der Dentalmaterialien. Aufgrund der guten Monomerlöslichkeit werden die Übergangsmetalle vorzugsweise in Form ihrer Acetylacetonate, 2-Ethylhexanoate oder THF-Addukte eingesetzt. Bevorzugt sind weiterhin ihre Komplexe mit mehrzähnigen Liganden wie z.B. 2-(2-Aminoethylamino)ethanol, Triethylentetramin, Dimethylglyoxim, 8-Hydroxychinolin, 2,2'-Bipyridin oder 1,10-Phenanthrolin. Ein erfindungsgemäß besonders geeigneter Initiator ist eine Mischung von Cumolhydroperoxid (CHP) mit mindestens einem der oben genannten Thioharnstoffderivate und Kupfer(II)-acetylacetonat. Erfindungsgemäß sind Zusammensetzungen bevorzugt, die keine Vanadiumverbindungen enthalten.

Die erfindungsgemäßen Zusammensetzungen enthalten vorzugsweise keine Barbitursäure oder Barbitursäure-Derivate wie 1,3,5-Trimethylbarbitursäure, 1-Benzyl-5-phenylbarbitursäure, 5-Butylbarbitursäure oder 1-Cylohexyl-5-ethylbarbitursäure. Zusammensetzungen, die Barbiturate enthalten, weisen eine unbefriedigende Lagerstabilität auf, weil Barbiturate durch Oxidation mit Luftsauerstoff polymerisationsauslösende Radikale bilden. Außerdem haben Barbiturate nachteilige physiologische Wirkungen wie Bradykardie, Hypotonie oder Blutstörungen.

Die erfindungsgemäßen Zusammensetzungen enthalten mindestens einen anorganischen Füllstoff. Füllstoffhaltige Zusammensetzungen werden als Komposite bezeichnet. Bevorzugt sind Zusammensetzungen, die mindestens einen Fluoroaluminiumsilikatglasfüllstoff (FAS- Füller) und/oder einen röntgenopaken Glasfüllstoff enthalten.

Bevorzugte röntgenopake Glasfüllstoffe weisen die folgende Zusammensetzung auf (Gew.-%): SiO₂: 20-80; B₂O₃: 2-15, BaO oder SrO: 0-40; Al₂O₃: 2-20; CaO und/oder MgO: 0-20; Na₂O, K₂O, Cs₂O: je 0-10; WO₃: 0-20; ZnO: 0-20; La₂O₃: 0-10; ZrO₂: 0-15; P₂O₅: 0-30; Ta₂O₅, Nb₂O₅ oder Yb₂O₃: 0-5; und CaF₂ und/oder SrF₂ 0-10. Besonders bevorzugt sind röntgenopake Glasfüller mit der Zusammensetzung (Gew.-%): SiO₂: 50-75; B₂O₃: 2-15; BaO oder SrO: 2-35; Al₂O₃: 2-15; CaO und/oder MgO: 0-10; und Na₂O: 0-10.

Besonders bevorzugte FAS-Füller haben die folgende Zusammensetzung (Gew.-%): SiO₂: 20-35; Al₂O₃: 15-35; BaO oder SrO: 10-25; CaO: 0-20; ZnO: 0-15; P₂O₅: 5-20; Na₂O, K₂O, Cs₂O: je 0-10; und CaF₂: 0,5-20. Besonders bevorzugt sind FAS-Füller mit der Zusammensetzung (Gew.-%): SiO₂: 20-30; Al₂O₃: 20-30; BaO oder SrO: 10-25; CaO: 5-20; P₂O₅: 5-20; Na₂O: 0-10; und CaF₂: 5-20.

Alle Angaben beziehen sich auf die Gesamtmasse des Glases, wobei alle Komponenten mit Ausnahme von Fluor als Oxide berechnet werden, so wie es bei Gläsern und Glaskeramiken üblich ist.

Die FAS-Füller und röntgenopaken Glasfüller haben vorzugsweise eine mittlere Partikelgröße von 0,2 bis 20 µm und besonders bevorzugt von 0,4 bis 5 µm.

Die erfindungsgemäßen Zusammensetzungen enthalten vorzugsweise 25 Gew.-% bis 80 Gew.-%, besonders bevorzugt 30 bis 75 Gew.-% und ganz besonders bevorzugt 40 bis 70 Gew.-% FAS-Füller und/oder röntgenopaken Glasfüller, jeweils bezogen auf die Gesamtmasse der Zusammensetzung.

Zusätzlich zu den genannten FAS- und röntgenopaken Glasfüllstoffen können die erfindungsgemäßen Zusammensetzungen weitere Füllstoffe enthalten.

Bevorzugte weitere Füllstoffe sind Metalloxide, besonders bevorzugt Mischoxide, die 60 bis 80 Gew.-% SiO₂ und mindestens eines der Metalloxide ZrO₂, Yb₂O₃, ZnO, Ta₂O₅, Nb₂O₅ und/oder La₂O₃ enthalten, so dass sich die Gesamtmenge auf 100 % ergänzt. Mischoxide wie SiO₂-ZrO₂ sind z.B. durch hydrolytische Cokondensation von Metallalkoxiden zugänglich. Die Metalloxide haben vorzugsweise eine mittlere Partikelgröße von 0,05 bis 10 µm, besonders bevorzugt von 0,1 bis 5 µm.

Weitere bevorzugte zusätzliche Füllstoffe sind pyrogene Kieselsäure oder Fällungskieselsäure mit einer Primärpartikelgröße von 0,01-0,15 µm sowie Quarz- oder Glaskeramikpulver mit einer Partikelgröße von 0,1 bis 15 µm, vorzugweise von 0,2 bis 5 µm, sowie Ytterbiumtrifluorid. Das Ytterbiumtrifluorid hat vorzugsweise eine Partikelgröße von 80 bis 900 nm und besonders bevorzugt 100 bis 300 nm.

Außerdem sind als weitere Füllstoffe sog. Kompositfüller bevorzugt. Diese werden auch als Isofüller bezeichnet. Hierbei handelt es sich um splitterförmige Polymerisate, die ihrerseits einen Füllstoff enthalten, vorzugsweise pyrogenes SiO₂ und/oder Ytterbiumtrifluorid wie oben definiert. Bevorzugt sind Polymerisate auf der Basis von Dimethacrylaten. Zur Herstellung der Isofüller werden der oder die Füllstoffe z.B. in eine Dimethacrylatharzmatrix eingearbeitet, die so erhaltene Kompositpaste anschließend thermisch polymerisiert und dann gemahlen.

Ein erfindungsgemäß bevorzugter Kompositfüller lässt sich beispielsweise durch thermische Härtung einer Mischung von Bis-GMA (8,80 Gew.-%), UDMA (6,60 Gew.- %), 1,10-Decandioldimethacrylat (5,93 Gew.-%), Dibenzoylperoxid + 2,6-Di-tert.-butyl-4-methylphenol (zusammen 0,67 Gew.-%), Glasfüller (mittlere Korngröße 0,4 µm; 53,0 Gew.-%) und YbF₃ (25,0 Gew.-%) und anschließendes Vermahlen des gehärteten Materials auf die gewünschte Korngröße herstellen. Alle Prozentangaben beziehen sich auf die Gesamtmasse des Kompositfüllers.

Als weitere Füllstoffe können auch sog. inertisierte Füllstoffe eingesetzt werden. Dabei handelt es sich um Glasfüllstoffe, deren Oberfläche mit einer Diffusionsbarriereschicht, z.B. auf Sol-Gel-Basis oder mit einer Polymerschicht, z.B. aus PVC, beschichtet ist. Bevorzugt sind die in der EP 2 103 296 A1 beschrieben Füllstoffe.

Zur Verbesserung des Verbundes zwischen Füllstoff und Matrix sind die Füllstoffe vorzugsweise mit methacrylat-funktionalisierten Silanen, wie z.B. 3-Methacryloyloxypropyltrimethoxysilan, oberflächenmodifiziert.

Die erfindungsgemäßen Zusammensetzungen enthalten vorzugsweise 0,1 bis 25 Gew.-%, bevorzugt 1 bis 20 Gew.-% und besonders bevorzugt 2 bis 15 Gew.-% eines oder mehrerer weiterer Füllstoffe, vorzugsweise eines oder mehrerer Metalloxide, pyrogene Kieselsäure und/oder Fällungskieselsäure, jeweils bezogen auf die Gesamtmasse der Zusammensetzung.

Die erfindungsgemäßen Zusammensetzungen können außerdem zusätzlich weitere Additive enthalten, vor allem Stabilisatoren, Farbmittel, mikrobizide Wirkstoffe, fluoridionenabgebende Additive, wie z.B. Fluorid-Salze, insbesondere NaF oder Ammoniumfluorid, oder Fluorsilane, optische Aufheller, Weichmacher und/oder UV-Absorber.

Die erfindungsgemäßen Zusammensetzungen enthalten vorzugsweise:
- 5 bis 60 Gew.-%, bevorzugt 8 bis 45 Gew.-% besonders bevorzugt 10 bis 35 Gew.-% mindestens eines radikalisch polymerisierbaren Monomers ohne Säuregruppe,
- 1 bis 15 Gew.-%, bevorzugt 2 bis 12 Gew.-% und besonders bevorzugt 3 bis 10 Gew.-% mindestens eines säuregruppenhaltigen, radikalisch polymerisierbaren Monomers,
- 25 bis 80 Gew.-%, bevorzugt 30 bis 75 Gew.-% und besonders bevorzugt 40 bis 70 Gew.-% mindestens eines FAS-Füllstoffs und/oder röntgenopaken Glasfüllstoffs,
- 0,1 bis 8 Gew.-%, bevorzugt 0,5 bis 6 Gew.-% und besonders bevorzugt 1 bis 5 Gew.-% mindestens eines Initiators für die radikalische Polymerisation und
- 0,5 bis 6,0 Gew.-%, bevorzugt 0,7 bis 5,0 Gew.-% und besonders bevorzugt 1,0 bis 4,0 Gew.-% mindestens eines Maskierungsmittels.

Wenn nicht anders angegeben, beziehen sich alle Prozentangaben hierin auf die Gesamtmasse der Zusammensetzung. Alle Mengenangaben für radikalisch polymerisierbare Monomere (poly- und monofunktionell) beziehen sich nur auf Monomere ohne Säuregruppe und schließen säuregruppenhaltige Monomere nicht ein.

Der Initiator kann ein Redoxinitiator, ein Photoinitiator oder ein Initiator für die duale Härtung sein. Die genannten Mengenangaben enthalten sämtliche Initiatorbestandteile, d.h. die Initiatoren selbst und ggf. Reduktionsmittel, Übergangsmetallverbindung etc. Erfindungsgemäß sind Zusammensetzungen bevorzugt, die mindestens einen Redoxinitiator oder mindestens einen Redoxinitiator und mindestens einen Photoinitiator enthalten.

Erfindungsgemäß sind solche Zusammensetzungen besonders bevorzugt, die die folgenden Bestandteile enthalten:
a) 0,5 bis 6 Gew.-%, bevorzugt 0,7 bis 5 Gew.-% und besonders bevorzugt 1,0 bis 4,0 Gew.-% mindestens eines festen Maskierungsmittels,
b) 5 bis 40 Gew.-%, bevorzugt 8 bis 30 Gew.-% besonders bevorzugt 10 bis 25 Gew.-% mindestens eines polyfunktionellen Monomers ohne Säuregruppe,
c) 1 bis 15 Gew.-%, bevorzugt 2 bis 12 Gew.-% und besonders bevorzugt 3 bis 10 Gew.-% mindestens eines säuregruppenhaltigen Monomers,
d) 0 bis 10 Gew.-%, bevorzugt 0 bis 8 Gew.-% und besonders bevorzugt 1 bis 5 Gew.-% einer oder mehrerer oligomerer Carbonsäuren,
e) 1 bis 20 Gew.-%, bevorzugt 2 bis 15 Gew.-% und besonders bevorzugt 3 bis 10 Gew.-% eines oder mehrerer monofunktioneller Monomere ohne Säuregruppe,
f) 25 bis 80 Gew.-%, bevorzugt 30 bis 75 Gew.-% und besonders bevorzugt 40 bis 70 Gew.-% mindestens eines FAS-Füllstoffs und/oder röntgenopaken Glasfüllstoffs,
g) 0,1 bis 25 Gew.-%, bevorzugt 1 bis 20 Gew.-% und besonders bevorzugt 2 bis 15 Gew.-% eines oder mehrerer zusätzlicher Füllstoffe,
h) 0,1 bis 8 Gew.-%, bevorzugt 0,5 bis 6 Gew.-% und besonders bevorzugt 1 bis 5 Gew.-% eines Initiators für die radikalische Polymerisation,
i) 0 bis 20 Gew.-%, bevorzugt 0,2 bis 10 Gew.-% und besonders bevorzugt 1 bis 7 Gew.-% Wasser und
j) 0,001 bis 5 Gew.-%, bevorzugt 0,002 bis 3 Gew.-% und besonders bevorzugt 0,005 bis 2 Gew.-% Additive, jeweils bezogen auf die Gesamtmasse der Zusammensetzung.

Zusammensetzungen, die einen Redoxinitiator enthalten, werden auch als selbsthärtend bezeichnet. Sie werden vorzugsweise in der Form von zwei räumlich getrennten Komponenten, d.h. als 2-Komponenten-System (2K System), eingesetzt. Oxidations-und Reduktionsmittel werden dabei in separate Komponenten der Zusammensetzung eingebarbeitet. Eine Komponente, die sog. Katalysatorpaste, enthält das Oxidationsmittel, vorzugsweise ein Peroxid oder Hydroperoxid, und die zweite Komponente, die sog. Basis-Paste, das entsprechende Reduktionsmittel und ggf. einen Photoinitiator und ggf. katalytische Mengen einer Übergansmetallverbindung. Die Polymerisation wird durch das Mischen der Komponenten gestartet. Zusammensetzungen, die sowohl einen Redox- als auch einen Photoinitiator enthalten, werden als dualhärtend bezeichnet.

Bei zweikomponentigen Zusammensetzungen wird das Maskierungsmittel bevorzugt der Komponente zugesetzt, in der sich das stark saure Haftmonomer, der FAS-Füllstoff und/oder der röntgenopake Glasfüllstoff befinden.

Erfindungsgemäß sind 2-Komponenten-Systeme bevorzugt. Sie sind vorzugsweise selbsthärtend oder dualhärtend. Die Pasten werden kurz vor der Anwendung vorzugsweise mit einer Doppelschubspritze miteinander gemischt.

Die Katalysatorpaste weist vorzugsweise die folgende Zusammensetzung auf:
a) 1,0 bis 12 Gew.-%, bevorzugt 1,4 bis 10 Gew.-% und besonders bevorzugt 2,0 bis 8,0 Gew.-% mindestens eines festen Maskierungsmittels,
b) 5 bis 40 Gew.-%, bevorzugt 8 bis 30 Gew.-%, besonders bevorzugt 10 bis 25 Gew.-% mindestens eines polyfunktionellen Monomers ohne Säuregruppe,
c) 2 bis 30 Gew.-%, bevorzugt 4 bis 24 Gew.-% und besonders bevorzugt 6 bis 20 Gew.-% mindestens eines säuregruppenhaltigen Monomers,
d) 0,1 bis 20 Gew.-%, bevorzugt 1 bis 16 Gew.-% und besonders bevorzugt 2 bis 10 Gew.-% einer oder mehrerer oligomerer Carbonsäuren,
e) 1 bis 20 Gew.-%, bevorzugt 2 bis 15 Gew.-% und besonders bevorzugt 3 bis 10 Gew.-% eines oder mehrerer monofunktioneller Monomere ohne Säuregruppe,
f) 25 bis 80 Gew.-%, bevorzugt 30 bis 75 Gew.-% und besonders bevorzugt 40 bis 70 Gew.-% mindestens eines FAS- und/oder Glasfüllstoffes,
g) 0,1 bis 25 Gew.-%, bevorzugt 1 bis 20 Gew.-% und besonders bevorzugt 2 bis 15 Gew.-% eines oder mehrerer zusätzlicher Füllstoffe,
h) 0,01 bis 16 Gew.-%, bevorzugt 0,02 bis 12 Gew.-% und besonders bevorzugt 0,03 bis 10 Gew.-% mindestens eines Peroxids und/oder Hydroperoxids und ggf. mindestens eines Photoinitiators,
i) 0 bis 20 Gew.-%, bevorzugt 0,2 bis 10 Gew.-% und besonders bevorzugt 1 bis 7 Gew.-% Wasser und
j) 0,001 bis 5 Gew.-%, bevorzugt 0,002 bis 3 Gew.-% und besonders bevorzugt 0,005 bis 2 Gew.-% Additive, jeweils bezogen auf die Gesamtmasse der Katalysatorpaste.

Die Basis-Paste weist vorzugsweise folgende Zusammensetzung auf:
a) entfällt,
b) 5 bis 40 Gew.-%, bevorzugt 8 bis 30 Gew.-% besonders bevorzugt 10 bis 25 Gew.-% mindestens eines polyfunktionellen Monomers ohne Säuregruppe,
c) entfällt,
d) entfällt,
e) 1 bis 20 Gew.-%, bevorzugt 2 bis 15 Gew.-% und besonders bevorzugt 3 bis 10 Gew.-% eines oder mehrerer monofunktioneller Monomere ohne Säuregruppe,
f) 25 bis 80 Gew.-%, bevorzugt 30 bis 75 Gew.-% und besonders bevorzugt 40 bis 70 Gew.-% mindestens eines Fluoroaluminiumsilikatglasfüllstoffs und/oder Glasfüllstoffs,
g) 0,1 bis 25 Gew.-%, bevorzugt 1 bis 20 Gew.-% und besonders bevorzugt 2 bis 15 Gew.-% eines oder mehrerer zusätzlicher Füllstoffe,
h) 0,01 bis 16 Gew.-%, bevorzugt 0,02 bis 12 Gew.-% und besonders bevorzugt 0,03 bis 10 Gew.-% mindestens eines geeigneten Reduktionsmittels und ggf. eines Photoinitiators,
i) 0 bis 20 Gew.-%, bevorzugt 0,2 bis 10 Gew.-% und besonders bevorzugt 1 bis 7 Gew.-% Wasser und
j) 0,001 bis 5 Gew.-%, bevorzugt 0,002 bis 3 Gew.-% und besonders bevorzugt 0,005 bis 2 Gew.-% Additive, jeweils bezogen auf die Gesamtmasse der Basis-Paste.

Zur Anwendung werden die Katalysator- und Basis-Paste vorzugsweise in etwa gleichen Anteilen miteinander gemischt. Sie eignen sich daher besonders zur Anwendung mit einer Doppelschubspritze.

Doppelschubspritzen weisen zwei getrennte zylindrische Kammern zur Aufnahme von Basis- und Katalysatorpaste auf. Die Komponenten werden mit zwei miteinander verbundenen Kolben gleichzeitig aus den Kammern herausgedrückt und vorzugsweise durch eine Mischkanüle gepresst und darin miteinander gemischt. Zum Herausdrücken der Pasten kann die Spritze in einen sogenannten Handdispenser eingesetzt werden, der die Handhabung der Spritzen erleichtert.

Die erfindungsgemäßen Zusammensetzungen zeichnen sich durch eine hohe Lagerstabilität und eine verbesserte Transparenz, vorzugsweise größer 10 %, und gute Selbsthaftung auf Schmelz/Dentin aus. Sie eignen sich besonders als Dentalwerkstoffe zur intraoralen Anwendung durch den Zahnarzt zur Restauration geschädigter Zähne (therapeutische Anwendung), insbesondere als dentale Zemente, Beschichtungs- oder Verblendmaterialien, Füllungskomposite und ganz besonders als Befestigungszemente. Die Transparenz wird auf die in den Ausführungsbeispielen beschriebene Weise bestimmt.

Zur Behandlung geschädigter Zähne werden diese vorzugsweise in einem ersten Schritt durch den Zahnarzt präpariert. Anschließend wird mindestens eine erfindungsgemäße Zusammensetzung auf oder in den präparierten Zahn appliziert. Danach kann die Zusammensetzung direkt gehärtet werden, vorzugsweise durch Bestrahlen mit Licht einer geeigneten Wellenlänge, beispielsweise bei der Versorgung von Kavitäten. Alternativ wird eine Dentalrestauration, beispielsweise ein Inlay, Onlay, Veneer, eine Krone, Brücke, ein Gerüst oder eine Dentalkeramik, in den präparierten Zahn eingebracht oder darauf aufgebracht. Die anschließende Härtung der Zusammensetzung erfolgt vorzugsweise durch Licht und/oder durch Selbsthärtung. Die Dentalrestauration wird hierbei am Zahn befestigt.

Die erfindungsgemäßen Zusammensetzungen können auch als extraorale Werkstoffe (nicht therapeutisch) eingesetzt werden, beispielsweise bei der Herstellung oder Reparatur von Dentalrestaurationen. Sie eignen sich zudem als Materialien zur Herstellung und Reparatur von Inlays, Onlays, Kronen oder Brücken.

Zur Herstellung von Dentalrestaurationen wie Inlays, Onlays, Kronen oder Brücken, wird mindestens eine erfindungsgemäße Zusammensetzung auf an sich bekannte Weise zu der gewünschten Dentalrestauration geformt und dann gehärtet. Die Härtung kann durch Licht, Selbsthärtung oder vorzugsweise thermisch erfolgen.

Bei der Reparatur von Dentalrestaurationen werden die erfindungsgemäßen Zusammensetzungen auf die zu reparierende Restauration aufgebacht, beispielsweise um Lücken auszubessern oder um Bruchstücke zu verkleben, und anschließend gehärtet.

Die Erfindung wird im Folgenden anhand von Figuren und Beispielen näher erläutert.
Figur 1 zeigt die Abnahme der Konzentration des sauren Monomers MDP in Abhängigkeit von der Lagerzeit in Kompositpasten mit (▼; ●) und ohne (■) EDTA.
Figur 2 zeigt die Abnahme der Scherhaftfestigkeit von Kompositzementen mit (▼; ●) und ohne (■) EDTA in Abhängigkeit von der Lagerzeit.

### Beispiel 1

### Untersuchung der Lagerstabilität von selbsthaftenden Kompositen mit/ohne EDTA

Es wurden die Kompositpasten K-1 bis K-3 mit den in Tabelle 1 angegebenen Zusammensetzungen (alle Angaben in Gew.-%) aus folgenden Komponenten hergestellt: Experimenteller röntgenopaker Glasfüllstoff 1 (Zusammensetzung (Gew.-%): Al₂O₃: 6; B₂O₃: 5; Na₂O: 8; CaO, BaO, K₂O: je ca. 2-3; CaF₂; MgO je ca. 1; und SiO₂: 70; unsilanisiert), 10-Methacryloyloxydecyldihydrogenphosphat (MDP, Orgentis), Triethylenglycoldimethacrylat (TEGDMA), NK-Ester 9G (Polyethylenglyco-400-dimethacrylat, Kowa Europa GmbH), V-392 (N,N'-Diethyl-1,3-bis(acrylamido)-propan, Ivoclar Vivadent AG), BHT (2,6-Di-*tert*-butyl-*p*-kresol): deionisiertes Wasser und Ethylendiamintetraessigsäure (EDTA, Aldrich).

**Tabelle 1: Zusammensetzung von Kompositpasten (Gew.-%)**

| **Bestandteil** | **K-1** *⁾ **(ohne EDTA)** | **K-2** | **K-3** |
|---|---|---|---|
| TEGDMA | 11,28 | 10,86 | 11,13 |
| NK-Ester 9G | 2,38 | 2,29 | 2,35 |
| V-392 | 14,66 | 14,12 | 14,47 |
| MDP | 4,20 | 4,04 | 4,14 |
| BHT | 0,04 | 0,04 | 0,04 |
| Wasser | 6,99 | 6,74 | 6,91 |
| EDTA | 0 | 3,66 | 1,26 |
| Glasfüller 1 | 60,45 | 58,25 | 59,70 |

| | | | |
|---|---|---|---|
| *⁾ Vergleichsbeispiel | | | |

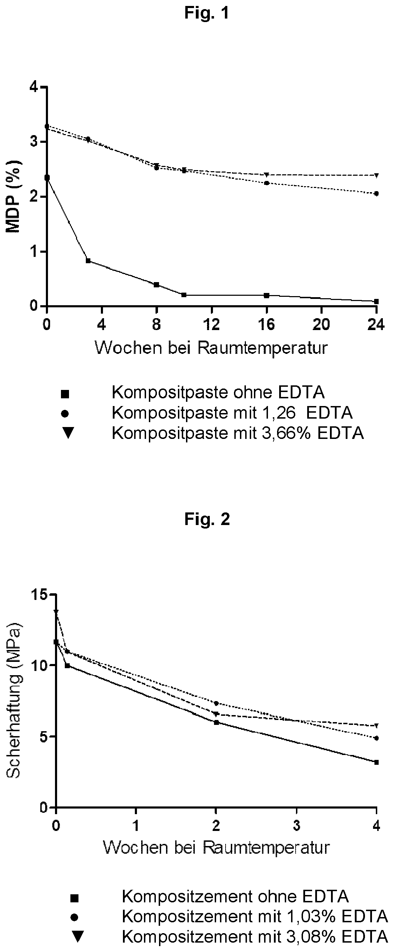

Die Pasten K-1 (ohne EDTA) sowie K-2 (3,66 % EDTA) und K-3 (1,26 % EDTA) wurden bei Raumtemperatur gelagert und im Abstand von einigen Wochen der Gehalt an MDP mittels HPLC bestimmt. Für die HPLC-Messung wurde ein Gerät HPLC UItimate 3000 (ThermoFisher Scientific) mit einer Säule 125x4 Nucleodur 100-5 C18ec mit UV/VIS-Detektor (220 nm) verwendet. Dabei wurde die Probe in Methanol gelöst und mit 0,01 mol/l H₃PO₄ in Wasser (A), Methanol (B) und Acetonitril (C) gemäß dem folgenden Programm eluiert. Die Ergebnisse sind in Fig. 1 dargestellt.

**Gradientenproaramm:**

| Zeit (min) | % A | % B | % C |
|---|---|---|---|
| 0 | 40 % | 40 % | 20 % |
| 6 | 40% | 40% | 20% |
| 20 | 0 % | 0 % | 100 % |
| 24 | 0% | 0% | 100% |

Die in Fig. 1. dargestellten Ergebnisse belegen eine deutlich verbesserte Lagerstabilität der Kompositpasten K-2 und K-3 mit EDTA. Die Kompositpaste **K-1** (■) ohne EDTA zeigt eine deutliche Abnahme des verfügbaren MDP schon nach 3-4 Wochen, während in den Kompositpasten **K-2 (▼)** und **K-3 (●)** mit EDTA auch nach 24 Wochen noch mehr als 2/3 des ursprünglichen MDP verfügbar war.

### Beispiel 2

### Herstellung von dualhärtenden selbsthaftenden Kompositzementen

Analog zu Beispiel 1 wurden die Katalysatorpasten KP-1 (ohne EDTA), KP-2 und KP-3 (mit EDTA) sowie die Basispaste mit den in Tabelle 2 angegebenen Zusammensetzungen hergestellt. Es wurde die Dentinhaftung der Materialien in Abhängigkeit von der Lagerzeit bestimmt. Für die Untersuchung der Dentinhaftung wurden Rinderzähne so in Kunststoffzylinder eingebettet, dass sich das Dentin und der Kunststoff in einer Ebene befanden. Nach 15 s Ätzung mit 37 %-iger Phosphorsäure wurde gründlich mit Wasser abgespült. Durch die Säureätzung wurden die Dentintubuli geöffnet. Dann wurden die Katalysatorpasten getrennt jeweils im Verhältnis 1:1 mit der Basispaste zu Zementen gemischt und anschließend mit einem Microbrush eine Schicht der zu testen Mischung auf den Zahn aufgetragen und 40 s mit einer Halogenlampe (Astralis 7, Ivoclar Vivadent AG) belichtet. Auf die Zementschicht wurde ein Kompositzylinder aus einem dentalen Kompositmaterial (Tetric^{®} Ceram; Ivoclar Vivadent AG) in zwei Schichten von je 1-2 mm, die jeweils durch 40 s Belichtung mit der Halogenlampe Astralis 7 gehärtete wurden, aufpolymerisiert. Anschließend wurden die Prüfkörper 24 h bei 37 °C in Wasser gelagert und die Scherhaftfestigkeit bestimmt. Die Ergebnisse sind in Fig. 2 dargestellt.

Mit dem Zement auf Basis der Basispaste und der Paste **KP-1 (■)** ohne EDTA wurde nach 28 Tagen ein Wert der Dentinhaftung von 3,21 MPa bestimmt. Demgegenüber ergaben die Zemente auf Basis der Basispaste und der Pasten mit EDTA **KP-2 (▼)** bzw. **KP-3 (●)** nach 4 Wochen Lagerung bei Raumtemperatur deutlich höhere Werte der Dentinhaftung von 5,76 MPa **(KP-2)** bzw. 4,42 MPa **(KP-3).**

**Tabelle 2: Zusammensetzung der Basis- und Katalysatorpasten (Gew.-%)**

| **Bestandteil** | **KP-1***⁾ | **KP-2** | **KP-3** | **Basispaste** |
|---|---|---|---|---|
| MDP | 3,39 | 3,39 | 3,39 | - |
| TEGDMA | 10,66 | 10,66 | 10,66 | 8,81 |
| NK-Ester 9G | 2,37 | 2,37 | 2,37 | 2,59 |
| UDMA | 14,59 | 14,59 | 14,59 | 12,90 |
| Dibenzoylperoxid (50 %) | 0,960 | 0,960 | 0,960 | - |
| N,N-Diethyl-3,5-di-tert.butyl-anilin | - | - | - | 1,00 |
| Campherchinon | - | - | - | 0,078 |
| 4-(Dimethylamino)-benzoesäureethylester | - | - | - | 0,155 |
| BHT | 0,0312 | 0,0312 | 0,0312 | - |
| Inhibitor¹⁾ | - | - | - | 0,00091 |
| EDTA | - | 3,08 | 1,03 | - |
| FAS-Füller (G018-056 1,0 µm, 5% silan.)²⁾ | 67,00 | 63,92 | 65,97 | 3,07 |
| FAS-Füller (G018-056 7,0 µm, 5% silan.)³⁾ | - | - | - | 69,70 |
| HDK 2000⁴⁾ | 1,00 | 1,00 | 1,00 | 1,49 |
| Benzyltributylammoniumchlorid | - | - | - | 0,207 |
| Farbpigment Lumilux LZ Flu Blau⁵⁾ | - | - | - | 0,00085 |

| | | | | |
|---|---|---|---|---|
| *⁾ Vergleichsbeispiel ¹⁾ TEMPO: 2,2,6,6-Tetramethylpiperidinyloxyl, CAS-Nummer 2564-83-2 ²⁾ Zusammensetzung (Gew.-%): Al₂O₃: 24; SiO₂: 23; CaO: 16,5; CaF₂: 16; BaO: 11,5; P₂O₅: 8; Na₂O: 2; 5% Silan; Mittlerer Partikeldurchmesser 1 µm (Schott AG, Mainz) ³⁾ Zusammensetzung (Gew.-%): Al₂O₃: 24; SiO₂: 23; CaO: 16,5; CaF₂: 16; BaO: 11,5; P₂O₅: 8; Na₂O: 2; 5% Silan; gMittlerer Partikeldurchmesser 7 µm (Schott AG, Mainz) ⁴⁾ pyrogene Kieselsäure; Trimethylsiloxy-Oberflächenmodifizierung; BET-Oberfläche (DIN ISO 9277 DIN 66132) unsilanisiert: ca. 200 m²/g; Dichte (SiO₂; DIN 51757): 2,2 g/cm³; Restsilanol-Gehalt (relativer Silanol-Gehalt bezogen auf unsilanisierte Kieselsäure mit ca. 2 SiOH/nm²): 25 % (Wacker Chemie AG) ⁵⁾ 2,5-Dihydroxyterephthalsäurediethylester (Riedel-de Haën AG) | | | | |

Die Transparenz der Kompositzemente wurde mittels eines Spektrophotometers (Konika-Minolta Spektrophotometer CM-5) an 1 mm dicken, auf Hochglanz polierten Prüfkörpern in Transmission gemessen. Die Transparenzbestimmung der Zemente auf der Basis der Basispaste und der Katalysatorpasten KP-1 bis KP-3 ergab die folgenden Transparenzwerte: **KP-1:** 21,1%; **KP-2:** 16,7%, **KP-3:** 18,8 %. Obwohl die Transparenz der Zemente auf Basis der Katalysatorpasten KP-2 und KP-3 im Vergleich zu dem Zement auf der Basis von KP-1 durch die Zugabe von EDTA etwas verringert wurde, sind die Transparenzwerte der erfindungsgemäßen Kompositzemente deutlich höher als die klassischer Glasionomerzemente, wie z.B. von Vivaglass CEM PL (Ivoclar Vivadent AG) mit einen Transparenz von 6,7 %.

## Patentansprüche

1. Radikalisch polymerisierbare Zusammensetzung, die mindestens ein saures, radikalisch polymerisierbares Monomer, mindestens einen Fluoroaluminiumsilikatglasfüllstoff und/oder röntgenopaken Glasfüllstoff und mindestens ein Maskierungsmittel enthält, **dadurch gekennzeichnet, dass** das Maskierungsmittel in fester Form vorliegt.

2. Zusammensetzung nach Anspruch 1, bei der das Maskierungsmittel aus Ethylendiamintetraessigsäure (EDTA) und deren Dinatrium-Salz (Dinatriumethylendiamin-tetraacetat), Nitrilotriessigsäure, Diethylentriaminpentaessigsäure, Tetranatriumiminodisuccinat und dem Trinatriumsalz der Methylglycindiessigsäure ausgewählt ist.

3. Zusammensetzung nach Anspruch 1 oder 2, bei der das Maskierungsmittel in partikulärer Form vorliegt und vorzugsweise eine volumenmittlere Partikelgröße (D50-Wert) von 300 bis 350 µm, besonders bevorzugt von 200 bis 250 µm und ganz besonders bevorzugt von 100 bis 170 µm und vorzugsweise auch einen D10-Wert von 130 bis 150 µm, besonders bevorzugt 80 bis 100 µm und ganz besonders bevorzugt 70 bis 55 µm aufweist.

4. Zusammensetzung nach einem der Ansprüche 1 bis 3, die
- 5 bis 60 Gew.-%, bevorzugt 8 bis 45 Gew.-% besonders bevorzugt 10 bis 35 Gew.-% mindestens eines radikalisch polymerisierbaren Monomers ohne Säuregruppe,
- 1 bis 15 Gew.-%, bevorzugt 2 bis 12 Gew.-% und besonders bevorzugt 3 bis 10 Gew.-% mindestens eines säuregruppenhaltigen, radikalisch polymerisierbaren Monomers,
- 25 bis 80 Gew.-%, bevorzugt 30 bis 75 Gew.-% und besonders bevorzugt 40 bis 70 Gew.-% mindestens eines Fluoroaluminiumsilikatglasfüllstoffs und/oder röntgenopaken Glasfüllstoffs,
- 0,1 bis 8 Gew.-%, bevorzugt 0,5 bis 6 Gew.-% und besonders bevorzugt 1 bis 5 Gew.-% mindestens eines Initiators für die radikalische Polymerisation und
- 0,5 bis 6,0 Gew.-%, bevorzugt 0,7 bis 5,0 Gew.-% und besonders bevorzugt 1,0 bis 4,0 Gew.-% mindestens eines Maskierungsmittels enthält, jeweils bezogen auf die Gesamtmasse der Zusammensetzung.

5. Zusammensetzung nach Anspruche 4, die
a) 0,5 bis 6 Gew.-%, bevorzugt 0,7 bis 5 Gew.-% und besonders bevorzugt 1,0 bis 4,0 Gew.-% mindestens eines festen Maskierungsmittels,
b) 5 bis 40 Gew.-%, bevorzugt 8 bis 30 Gew.-% besonders bevorzugt 10 bis 25 Gew.-% mindestens eines polyfunktionellen Monomers ohne Säuregruppe,
c) 1 bis 15 Gew.-%, bevorzugt 2 bis 12 Gew.-% und besonders bevorzugt 3 bis 10 Gew.-% mindestens eines säuregruppenhaltigen Monomers,
d) 0 bis 10 Gew.-%, bevorzugt 0 bis 8 Gew.-% und besonders bevorzugt 1 bis 5 Gew.-% einer oder mehrerer oligomerer Carbonsäuren,
e) 1 bis 20 Gew.-%, bevorzugt 2 bis 15 Gew.-% und besonders bevorzugt 3 bis 10 Gew.-% eines oder mehrerer monofunktioneller Monomere ohne Säuregruppe,
f) 25 bis 80 Gew.-%, bevorzugt 30 bis 75 Gew.-% und besonders bevorzugt 40 bis 70 Gew.-% mindestens eines Fluoroaluminiumsilikatglasfüllstoffs s und/oder röntgenopaken Glasfüllstoffs,
g) 1 bis 25 Gew.-%, bevorzugt 1 bis 20 Gew.-% und besonders bevorzugt 2 bis 15 Gew.-% eines oder mehrerer zusätzlicher Füllstoffe,
h) 0,1 bis 8 Gew.-%, bevorzugt 0,5 bis 6 Gew.-% und besonders bevorzugt 1 bis 5 Gew.-% eines Initiators für die radikalische Polymerisation,
i) 0 bis 20 Gew.-%, bevorzugt 0,2 bis 10 Gew.-% und besonders bevorzugt 1 bis 7 Gew.-% Wasser und
j) 0,001 bis 5 Gew.-%, bevorzugt 0,002 bis 3 Gew.-% und besonders bevorzugt 0,005 bis 2 Gew.-% Additive enthält, jeweils bezogen auf die Gesamtmasse der Zusammensetzung.

6. Zusammensetzung nach Anspruch 4 oder 5, die als polyfunktionelles Monomer (b) mindestens ein Monomer enthält, das aus Bisphenol-A-dimethacrylat, Bis-GMA (ein Additionsprodukt aus Methacrylsäure und Bisphenol-A-diglycidylether), ethoxy- oder propoxyliertes Bisphenol-A-dimethacrylaten, wie Bisphenol-A-dimethacrylat mit 3 Ethoxygruppen oder 2,2-Bis[4-(2-methacryl-oyloxypropoxy)phenyl]propan, Urethanen aus 2-(Hydroxymethyl)acrylsäure und Diisocyanten, wie 2,2,4-Trimethylhexamethylendiisocyanat oder Isophorondiisocyanat, UDMA (ein Additionsprodukt aus 2-Hydroxyethylmethacrylat und 2,2,4-Trimethylhexamethylen-1,6-diisocyanat), Tetra-methylxylylendiurethanethylenglycoldi(meth)acrylat bzw. Tetramethylxylylen-diurethan-2-methylethylenglykoldiurethandi(meth)acrylat (V380), Di-, Tri- oder Tetraethylenglycoldimethacrylat, Trimethylolpropantrimethacrylat, Pentaerythrittetramethacrylat sowie Glycerindi- und -trimethacrylat, 1,4-Butandioldi-methacrylat, 1,10-Decandioldimethacrylat (D₃MA), Bis(methacryloyloxy-methyl)tricyclo-[5.2.1.0^{2,6}]decan (DCP), Polyethylenglycol- oder Polypropylen-glycoldimethacrylaten, wie Polyethylenglycol-200-dimethacrylat (PEG-200-DMA) oder Polyethylenglycol-400-dimethacrylat (PEG-400-DMA), 1,12-Dodecandioldimethacrylat, radikalisch polymerisierbaren Pyrrolidonen, wie z.B. 1,6-Bis(3-vinyl-2-pyrrolidonyl)-hexan, Bisacrylamiden, wie Methylen- oder Ethylenbisacrylamid, Bis(meth)acrylamiden, wie N,N'-Diethyl-1,3-bis(acrylamido)-propan, 1,3-Bis(methacrylamido)propan, 1,4-Bis(acrylamido)-butan oder 1,4-Bis(acryloyl)-piperazin, und Mischungen davon ausgewählt ist.

7. Zusammensetzung nach einem der Ansprüche 1 bis 6, die als säuregruppenhaltiges Monomer (c) mindestens ein Monomer enthält, das aus Monomeren, die eine Phosphorsäureestergruppe oder Phosphonsäuregruppe enthalten, vorzugsweise 2-Methacryloyloxyethylphenyl-hydrogenphosphat, 10-Methacryloyloxydecyl-dihydrogenphosphat (MDP) Glycerindimethacrylatdihydrogenphosphat, Dipentaerythritpentamethacryloyloxyphosphat. 4-Vinylbenzylphosphonsäure, 2-[4-(Dihydroxyphosphoryl)-2-oxa-butyl]-acrylsäure und/oder 2-[4-(Dihydroxyphosphoryl)-2-oxa-butyl]-acrylsäure-2,4,6-trimethylphenylester, und/oder Monomeren ausgewählt ist, die eine Carboxygruppe enthalten, vorzugsweise 4-(Meth)acryloyloxyethyltrimellitsäureanhydrid, 10-Methacryloyloxydecylmalonsäure, N-(2-Hydroxy-3-methacryloyloxypropyl)-N-phenylglycin und/oder 4-Vinylbenzoesäure.

8. Zusammensetzung nach einem der Ansprüche 4 bis 7, die als oligomere Carbonsäure (d) Polyacrylsäure mit einer zahlenmittleren Molmasse kleiner 7200 g/mol, bevorzugt kleiner 7000 g/mol und besonders bevorzugt kleiner 6800 g/mol enthält.

9. Zusammensetzung nach einem der Ansprüche 4 bis 8, die als monofunktionelles Monomer (e) mindestens ein Monomer enthält, das aus Benzyl-, Tetrahydrofurfuryl(meth)acrylat, Isobornyl(meth)acrylat, p-Cumyl-phenoxyethylenglycolmethacrylat (CMP-1E) und 2-([1,1'-Biphenyl]-2-oxy)ethylmethacrylat (MA-836), Tricyclodecanmethyl(meth)acrylat, 2-(2-Biphenyloxy)ethyl(meth)-acrylat, 2-Hydroxyethyl(methacrylat), Hydroxyethylpropyl(methacrylat), 2-Acet-oxyethylmethacrylat und Mischungen davon ausgewählt ist.

10. Zusammensetzung nach einem der Ansprüche 4 bis 9, die einen röntgenopaken Glasfüllstoff mit der Zusammensetzung (Gew.-%): SiO₂: 20-80; B₂O₃: 2-15, BaO oder SrO: 0-40; Al₂O₃: 2-20; CaO und/oder MgO: 0-20; Na₂O, K₂O, Cs₂O: je 0-10; WO₃: 0-20; ZnO: 0-20; La₂O₃: 0-10; ZrO₂: 0-15; P₂O₅: 0-30; Ta₂O₅, Nb₂O₅ oder Yb₂O₃: 0-5; und CaF₂ und/oder SrF₂ 0-10; oder vorzugsweise SiO₂: 50-75; B₂O₃: 2-15; BaO oder SrO: 2-35; Al₂O₃: 2-15; CaO und/oder MgO: 0-10; und Na₂O: 0-10; und/oder einen Fluoroaluminiumsilikatglasfüllstoff mit der Zusammensetzung (Gew.-%): SiO₂: 20-35; Al₂O₃: 15-35; BaO oder SrO: 10-25; CaO: 0-20; ZnO: 0-15; P₂O₅: 5-20; Na₂O, K₂O, Cs₂O: je 0-10; und CaF₂: 0,5-20 Gew.-%; oder vorzugsweise SiO₂: 20-30; Al₂O₃: 20-30; BaO oder SrO: 10-25; CaO: 5-20; P₂O₅: 5-20; Na₂O: 0-10; und CaF₂: 5-20 Gew.-% enthält, wobei sich alle Angaben auf die Gesamtmasse des Glases beziehen und wobei alle Komponenten mit Ausnahme von Fluor als Oxide berechnet werden.

11. Zusammensetzung nach einem der Anspruche 1 bis 10, die mindestens einen Redoxinitiator oder mindestens einen Redoxinitiator und mindestens einen Photoinitiator enthält.

12. Zusammensetzung nach einem der Ansprüche 4 bis 11, die eine Katalysatorpaste und eine Basis-Paste umfasst, wobei die Katalysatorpaste
a) 1,0 bis 12 Gew.-%, bevorzugt 1,4 bis 10 Gew.-% und besonders bevorzugt 2,0 bis 8,0 Gew.-% mindestens eines festen Maskierungsmittels,
b) 5 bis 40 Gew.-%, bevorzugt 8 bis 30 Gew.-%, besonders bevorzugt 10 bis 25 Gew.-% mindestens eines polyfunktionellen Monomers ohne Säuregruppe,
c) 2 bis 30 Gew.-%, bevorzugt 4 bis 24 Gew.-% und besonders bevorzugt 6 bis 20 Gew.-% mindestens eines säuregruppenhaltigen Monomers,
d) 0,1 bis 20 Gew.-%, bevorzugt 1 bis 16 Gew.-% und besonders bevorzugt 2 bis 10 Gew.-% einer oder mehrerer oligomerer Carbonsäuren,
e) 1 bis 20 Gew.-%, bevorzugt 2 bis 15 Gew.-% und besonders bevorzugt 3 bis 10 Gew.-% eines oder mehrerer monofunktioneller Monomere ohne Säuregruppe,
f) 25 bis 80 Gew.-%, bevorzugt 30 bis 75 Gew.-% und besonders bevorzugt 40 bis 70 Gew.-% mindestens eines Fluoroaluminiumsilikatglasfüllstoffs und/oder Glasfüllstoffes,
g) 0,1 bis 25 Gew.-%, bevorzugt 1 bis 20 Gew.-% und besonders bevorzugt 2 bis 15 Gew.-% eines oder mehrerer zusätzlicher Füllstoffe,
h) 0,01 bis 16 Gew.-%, bevorzugt 0,02 bis 12 Gew.-% und besonders bevorzugt 0,03 bis 10 Gew.-% mindestens eines Peroxids und/oder Hydroperoxids und ggf. mindestens eines Photoinitiators,
i) 0 bis 20 Gew.-%, bevorzugt 0,2 bis 10 Gew.-% und besonders bevorzugt 1 bis 7 Gew.-% Wasser und
j) 0,001 bis 5 Gew.-%, bevorzugt 0,002 bis 3 Gew.-% und besonders bevorzugt 0,0051 bis 2 Gew.-% Additive enthält, jeweils bezogen auf die Gesamtmasse der Katalysatorpaste.
und wobei die Basis-Paste
a) entfällt,
b) 5 bis 40 Gew.-%, bevorzugt 8 bis 30 Gew.-% besonders bevorzugt 10 bis 25 Gew.-% mindestens eines polyfunktionellen Monomers ohne Säuregruppe,
c) entfällt,
d) entfällt,
e) 1 bis 20 Gew.-%, bevorzugt 2 bis 15 Gew.-% und besonders bevorzugt 3 bis 10 Gew.-% eines oder mehrerer monofunktioneller Monomere ohne Säuregruppe,
f) 25 bis 80 Gew.-%, bevorzugt 30 bis 75 Gew.-% und besonders bevorzugt 40 bis 70 Gew.-% mindestens eines Fluoroaluminiumsilikatglasfüllstoffs und/oder Glasfüllstoffs,
g) 0,1 bis 25 Gew.-%, bevorzugt 1 bis 20 Gew.-% und besonders bevorzugt 2 bis 15 Gew.-% eines oder mehrerer zusätzlicher Füllstoffe,
h) 0,01 bis 16 Gew.-%, bevorzugt 0,02 bis 12 Gew.-% und besonders bevorzugt 0,03 bis 10 Gew.-% mindestens eines geeigneten Reduktionsmittels und ggf. eines Photoinitiators,
i) 0 bis 20 Gew.-%, bevorzugt 0,2 bis 10 Gew.-% und besonders bevorzugt 1 bis 7 Gew.-% Wasser und
j) 0,001 bis 5 Gew.-%, bevorzugt 0,002 bis 3 Gew.-% und besonders bevorzugt 0,0051 bis 2 Gew.-% Additive enthält, jeweils bezogen auf die Gesamtmasse der Basis-Paste.

13. Zusammensetzung nach Anspruch 12, wobei die Katalysatorpaste und die Basis-Paste jeweils in unterschiedlichen Kammern einer Doppelschubspritze enthalten sind.

14. Zusammensetzung nach einem der Ansprüche 1 bis 13 zur therapeutischen Anwendung als Dentalwerkstoff, vorzugsweise als dentaler Zement, Beschichtungs- oder Verblendmaterial, Füllungskomposit oder Befestigungszement.

15. Nicht-therapeutische Verwendung einer Zusammensetzung gemäß einem der Ansprüche 1 bis 13 zur Herstellung oder Reparatur von Dentalrestaurationen, insbesondere von Inlays, Onlays, Kronen oder Brücken.
